# EUROPEAN PATENT SPECIFICATION

(11) **EP 2 214 625 B1**
(45) Date of publication and mention of the grant of the patent: **03.10.2018**
(21) Application number: 08852468.1
(22) Date of filing: 21.11.2008
(51) Int. Cl.: A61K 8/27, A61K 8/29, A61K 8/31, A61K 8/49, A61K 8/63, A61K 8/64, A61K 8/67, A61Q 17/04, A61Q 19/08, A61K 8/60, A61K 8/36, A61K 8/34

(54) **EMULSION COMPOSITION**
EMULSIONSZUSAMMENSETZUNG
COMPOSITION D'ÉMULSION

(30) Priority: 22.11.2007 JP 2007302985
(43) Date of publication of application: 11.08.2010
(73) Proprietor: FUJIFILM Corporation, Tokyo 106-8620 (JP)
(72) Inventor: KUBO, Toshiaki, Ashigarakami-gun Kanagawa 258-8577 (JP); KINAI, Miki, Ashigarakami-gun Kanagawa 258-8577 (JP)
(74) Representative: Klunker IP Patentanwälte PartG mbB
(86) International application number: PCT/JP2008/071747
(87) International publication number: WO 2009/066799

(56) References cited:
- EP-A1- 1 287 807
- EP-A2- 0 559 319
- WO-A1-95/34278
- WO-A2-00/12053
- JP-A- 2002 308 752
- JP-A- 2002 308 752
- JP-A- 2004 026 741
- JP-A- 2007 270 073
- JP-A- 2007 270 073
- JP-A- 2007 284 359
- JP-A- 2008 239 609

## Description

### Technical Field

The present invention relates to an emulsion composition.

### Background Art

In recent years, it is found that active oxygen removal properties or antioxidative property of pigment components extracted from natural products, such as carotenoids, are effective in removing oxidation stress in the living body, and the application thereof to various fields, such as health food, pharmaceuticals for external use, and cosmetics, has been expected. However, the components have problems in stability and lightfastness since the components have high activity.

For example, astaxanthin, which is a kind of carotenoid, is a raw material whose activity has drawn attention, but its stability is low. Regarding a countermeasure to the problems, it is known that the stability is improved by, for example, a physical method such as a method of sealing the component in an oxygen impermeable wrapping material (e.g., Japanese Patent Application Laid-Open (JP-A) No. 8-12896) or a chemical method such as a method of using flavanol and kojic acid in combination (e.g., JP-A No. 8-332052) or a method of using an organic acid or organic acid salt together with gallic acid (e.g., JP-A No. 2002-218940).

In general, in order to protect the skin from the development of dry rough skin or wrinkles, sagging, pigmentation, etc., caused by regular exposure to ultraviolet rays, there is a proposal for including a ultraviolet shielding agent such as titanium oxide or zinc oxide (e.g., Japanese Patent No. 3863675). EP1287807 A1 discloses a titanium oxide- or zinc oxide-containing cosmetic composition capable of maintaining the emulsion stability for a long period of time by virtue of thickening of a polymer having a carboxyl group in the side chain.

However, the ultraviolet shielding agent (UV scattering agent) also has a catalytic effect. Therefore, when a composition includes an active oxygen remover or anti-oxidizing agent derived from a natural component together with the ultraviolet shielding agent, the stability of the active oxygen remover or anti-oxidizing agent as an active ingredient is decreased.

### DISCLOSURE OF INVENTION

Thus, an emulsion composition has been desired in which an active oxygen remover and/or anti-oxidizing agent as an active ingredient can be preserved with excellent stability over a long period of time even when a UV scattering agent is used in combination.

An object of the present invention is defined in claim 1.

According to the present invention, at least one of an active oxygen remover or an anti-oxidizing agent can be preserved with excellent stability even when a UV scattering agent is used in combination.

### BEST MODE FOR CARRYING OUT THE INVENTION

An emulsion composition of the present invention has an oil phase and an aqueous phase, and includes (A) a UV scattering agent and (B) at least one of an active oxygen remover or an anti-oxidizing agent. The UV scattering agent is added to the oil phase of the emulsion composition, and the at least one of an active oxygen remover or an anti-oxidizing agent is contained in O/W emulsion particles dispersed in an aqueous phase of the emulsion composition.

In the emulsion composition of the present invention, the UV scattering agent (sometimes referred to as component (A) in the present invention) and at least one selected from an active oxygen remover or an anti-oxidizing agent (sometimes referred to as component (B) in the present invention) are separately added to respectively different phases among the aqueous phase and oil phase of the emulsion composition. Thus, even though a UV scattering agent having a catalytic effect or the like is used in an emulsion composition containing at least one of an active oxygen remover or an anti-oxidizing agent, the at least one of an active oxygen remover or an anti-oxidizing agent can be favorably preserved without deteriorating the stability since the UV scattering agent is contained in a phase different from the phase containing the at least one of an active oxygen remover or an anti-oxidizing agent. As a result, an emulsion composition can be provided which can retain the functionality of the at least one of an active oxygen remover or an anti-oxidizing agent over a long period of time.

In the present specification, the separate addition of the respective components to respectively different phases of the emulsion composition (the aqueous phase and the oil phase) is sometimes referred to as "separate addition to different phases".

From the viewpoint of effectively demonstrating the function of the component (B), the component (B) is contained in the aqueous phase of the emulsion composition. The component (B) is contained in O/W (oil/water) emulsion particles that are dispersed in the aqueous phase of the emulsion composition. The component (A) is contained in the oil phase of the emulsion composition, which is an oil phase different from the O/W emulsion particles containing the component (B).

When the component (B) is blended in the emulsion composition of the present invention as O/W emulsion particles dispersed in the aqueous phase, it is preferable to add another emulsion composition (hereinafter sometimes referred to as a "water dispersion") in which the O/W emulsion particles containing the component (B) are dispersed, to the aqueous phase of the emulsion composition, whereby an emulsion composition in which fine O/W emulsion particles are dispersed in the aqueous phase can be obtained easily. In this case, each of the components described below may be contained in a phase of the emulsion composition or in a phase of the water dispersion prepared in advance for obtaining O/W emulsion particles containing the component (B), as long as the obtained configuration conforms to the configuration of the emulsion composition of the present invention.

### <UV scattering agent>

The UV scattering agent as the component (A) in the present invention is a compound, which is sometimes referred to as "ultraviolet shielding agent". Examples thereof include an inorganic powder and an organic compound, each of which has a function of reflecting or shielding ultraviolet rays.

Examples of the inorganic powder include powder of titanium oxide, zirconium oxide, cerium oxide, zinc oxide, or talc. Among the inorganic powder, powder of titanium oxide or zinc oxide is preferable from the viewpoint of protection against ultraviolet ray protection ability.

In order to increase the compatibility of the inorganic powder with the oil phase or aqueous phase of the emulsion composition, the inorganic powder may have been subjected to hydrophilizing surface treatment or hydrophobing surface treatment. Examples of the surface-treated inorganic powder include a surface-coated inorganic powder whose surface has been coated with oil, such as a silicone oil or a fluorine-containing oil; a fatty acid soap, such as silicic acid anhydride (silica), aluminum stearate, or zinc palmitate; a fatty acid, such as stearic acid, myristic acid, or palmitic acid; and a fatty acid ester, such as dextrin palmitate. The surface treatment to be conducted varies depending on the type of the inorganic powder to be treated and the phase of an emulsion composition to which the inorganic powder is to be added. When the inorganic powder is added to the oil phase, silicone treatment, alkyl fatty acid treatment, or the like may be preferably employed from the viewpoint of compatibility with the oil phase. When the inorganic powder is added to the aqueous phase, silica treatment is preferably employed from the viewpoint of dispersibility in water.

The inorganic powder UV scattering agent may be used singly or in combination of two or more thereof, in consideration of the compatibility with the phase to which the UV scattering agent is to be added.

Examples of the organic compound as a UV scattering agent include an organic dye, such as an azo dye, a xanthine dye, a quinoline dye, a triphenylmethane dye, or an anthraquinone dye; an organic pigment, such as lake, an azo pigment, an indigo pigment, or a phthalocyanine pigment; and high molecular weight fine particles, such as a polyethylene, polymethyl methacrylate, a styrene-alkyl methacrylate copolymer, a styrene-alkyl acrylate copolymer, laminated powder of polyethylene terephthalate and polymethyl methacrylate, and nylon powder. Furthermore, hollow particles and porous particles of such high molecular compounds are also usable. Among the organic UV scattering agents, a polymethyl methacrylate, a styrene-alkyl methacrylate copolymer, and a styrene-alkyl acrylate copolymer are preferable from the viewpoint of scattering properties, with a polymethyl methacrylate being particularly preferable. Moreover, hollow particles and porous particles of these high molecular compounds are also preferable since they are in a form by which the scattering properties as a UV scattering agent can be enhanced.

The content of the UV scattering agent in the emulsion composition of the present invention varies according to the type of the UV scattering agent to be used. From the viewpoint of ultraviolet ray shielding ability, the content of the UV scattering agent may be from 0.01 mass% to 20 mass%, and preferably from 0.1 mass% to 15 mass%, with respect to the mass of the entire emulsion composition.

### <Active oxygen remover>

The active oxygen remover as the component (B) in the present invention is a carotenoid.

Examples of the anti-oxidizing agent as the component (B) in the present invention include a vitamin C and a vitamin E. Examples of the vitamin C include ascorbic acid and derivatives thereof and salts thereof, such as L-ascorbic acid, L-ascorbyl palmitate, L-ascorbyl dipalmitate, L-ascorbyl isopalmitate, L-ascorbyl diisopalmitate, L-ascorbyl tetraisopalmitate, L-ascorbyl stearate, L-ascorbyl distearate, L-ascorbyl isostearate, L-ascorbyl diisostearate, L-ascorbyl myristate, L-ascorbyl dimyristate, L-ascorbyl isomyristate, L-ascorbyl diisomyristate, L-ascorbyl oleate, L-ascorbyl dioleate, L-ascorbyl 2-ethyl hexanoate, sodium L-ascorbyl 2-phosphate, potassium L-ascorbyl phosphate, magnesium L-ascorbyl phosphate, calcium L-ascorbyl phosphate, aluminum L-ascorbyl phosphate, sodium L-ascorbyl sulfate, potassium L-ascorbyl sulfate, magnesium L-ascorbyl sulfate, calcium L-ascorbyl sulfate, aluminum L-ascorbyl sulfate, sodium L-ascorbate, potassium L-ascorbate, magnesium L-ascorbate, calcium L-ascorbate, and aluminum L-ascorbate. Examples of the vitamin E include tocopherol and derivatives thereof and salts thereof, such as dl-α (β, γ)-tocopherol, dl-α-tocopherol acetate, dl-α-tocopherol nicotinate, dl-α-tocopherol linolate, and dl-α-tocopherol succinate, and ubiquinones;.

Moreover, it is possible to use one compound selected from the active oxygen removers and anti-oxidizing agents, or to use two or more compounds selected therefrom in combination. From the viewpoint of storage stability, it is preferable to use, in combination, at least one active oxygen remover and at least one anti-oxidizing agent, each of which may be selected from those described above.

Examples of carotenoids include actinioerythrin, astaxanthin, bixin, canthaxanthin, capsanthin, β-8'-apocarotenal, β-12'-apocarotenal, α-carotene, β-carotene, γ-carotene, β-cryptoxanthin, lutein, lycopine, violaxanthin, zeaxanthin, fucoxanthin, and derivatives thereof.

Among the above, β-carotene, astaxanthin, lutein, zeaxanthin, and β-cryptoxanthin are preferable. Particularly, astaxanthin, whose antioxidant effect, anti-inflammatory effect, anti-skin aging effect, and whitening effect have been recognized, is preferable.

The content of the component (B) in the emulsion composition of the present invention is preferably 0.00001 to 50 mass%, and more preferably 0.0001 to 30 mass%. When the content is within the above-mentioned range, the functions as the active oxygen remover and/or the anti-oxidizing agent can be effectively performed. When a plant extract is used, the dry solid content of the plant extract may be within the above-mentioned range.

### <UV absorber>

The emulsion composition of the present invention may contain a UV absorber for the improvement in ultraviolet ray protection ability. As a UV absorber which can be added to the emulsion composition of the present invention, a compound having an absorption maximum within at least one of the UV-A region (320 nm to 400 nm) or the UV-B region (290 nm to 320 nm) can be used. Examples thereof include cinnamic acid UV absorbers, such as 2-ethylhexyl para-methoxycinnamate, isopropyl para-methoxycinnamate, diethanolamine para-methoxyhydrocinnamate, glyceryl dipara-methoxycinnamate mono-2-ethylhexanoate, octyl methoxycinnamate, and diisopropyl methyl cinnamate; benzophenone UV absorbers, such as 2-hydroxy-4-methoxybenzophenone, 2-hydroxy-4-methoxybenzophenone-5-sulfuric acid, sodium 2-hydroxy-4-methoxybenzophenone-5-sulfate, 2,4-dihydroxybenzophenone, 2,2'-dihydroxy-4,4'-dimethoxybenzophenone, 2,2'-dihydroxy-4-methoxybenzophenone, 2,2',4,4'-tetrahydroxybenzophenone, 2-hydroxy-4-n-octoxybenzophenone; benzoic acid UV absorbers, such as diethylamino hydroxybenzoyl hexyl benzoate, para-aminobenzoic acid, ethyl para-aminobenzoate, butyl para-aminobenzoate, 2-ethylhexyl para-dimethylaminobenzoate, glyceryl para-aminobenzoate, and amyl para-aminobenzoate; salicylic acid UV absorbers, such as 2-ethylhexyl salicylate, triethanolamine salicylate, homomenthyl salicylate, dipropyleneglycol salicylate, methyl salicylate, ethyleneglycol salicylate, phenyl salicylate, amyl salicylate, benzyl salicylate, isopropylbenzyl salicylate, and potassium salicylate; dibenzoylmethane UV absorbers, such as 4-t-butyl-4'-methoxydibenzoylmethane, 4-isopropyldibenzoylmethane, 4-methoxydibenzoylmethane, and 4-t-butyl-4'-hydroxydibenzoylmethane; urocanic acid UV absorbers, such as ethyl urocanate; menthyl-o-amino benzoate, 2-phenyl-benzimidazole-5-sulfuric acid, 2-phenyl-5-methylbenzoxanol, 3-(4-methylbenzylidene) camphor, 2-ethylhexyl-2-cyano-3,3-diphenyl acrylate, 2-ethyl-2-cyano-3,3'-diphenyl acrylate, 2-(2'-hydroxy-5-methylphenyl) benzotriazole, menthyl anthranilate, and octocrylene.

From the viewpoint of ultraviolet ray protection, the content of the UV absorber in the emulsion composition of the present invention may be from 0.01 mass% to 20 mass%, and preferably from 0.1 mass% to 15 mass%, with respect to the total mass of the emulsion composition.

### <Emulsifier>

The emulsion composition of the present invention may contain a phospholipid and/or a surfactant as an emulsifier.

Specific examples of phospholipids usable in the emulsion composition of the present invention include lecithin, phosphatidic acid, bisphosphatidic acid, lecithin, phosphatidylethanolamine, phosphatidylmethylethanolamine, phosphatidylserine, phosphatidylinositol, phosphatidylglycerol, diphosphatidylglycerol, and sphingomyelin. Further examples include various lecithins such as
those derived from plants, such as soybean, corn, peanut, rape seed, and wheat, each containing such phospholipids ingredients;
those derived from animals, such as egg yolk and cow; and
those derived from microorganisms, such as Escherichia coli.

A lecithin that is a mixture of two or more of the above lecithins, and hydrogenated lecithins may also be used. The origins of the phospholipids are not limited. For example, phospholipids derived from vegetable oils such as soybean oil, and phospholipids derived from animals such as egg yolk, and the like are usable. Preferably, the phospholipid has been purified.

In the present invention, the scope of glycerophospholipids also includes glycerophospholipids having one fatty acid residue in a molecule as a result of enzymolysis, i.e., lysolecithins.

In the present invention, from the viewpoint of emulsion stability, lecithin is particularly preferable.

Examples of a commercially-available lecithin include LECION (tradename) series and LECIMAL EL (tradename) manufactured by Riken Vitamin Co., Ltd.

Products with a purity of lecithin of 60 mass% or higher are industrially used as lecithin. In the present invention, however, products having a purity of lecithin of 80 mass% or higher, which are generally referred to as "high purity lecithin", are preferable, and products having a purity of lecithin of 90 mass% or more are more preferable.

The phospholipid may be used singly or as a mixture of two or more thereof in the present invention.

In the emulsion composition of the present invention, the content of phospholipid varies depending on the form of the emulsion composition. In order to obtain a water dispersion in which fine O/W emulsion particles are dispersed, the content of phospholipid in the water dispersion is preferably from 0.001 mass% to 20 mass%, and more preferably from 0.001 to 10 mass%, relative to the water dispersion from the viewpoint of emulsion stability.

There is no limitation on a surfactant usable in the emulsion composition of the present invention, and a nonionic emulsifier is preferable. Examples of the nonionic emulsifier include glycerol fatty acid esters, organic acid monoglycerides, polyglycerol fatty acid esters, propyleneglycol fatty acid esters, polyglycerol-condensed ricinoleic acid esters, sorbitan fatty acid esters, and sucrose fatty acid esters. More preferable examples include polyglycerol fatty acid esters, sorbitan fatty acid esters, and sucrose fatty acid esters. The emulsifier does not need to have been highly purified by distillation or the like, and may be a reaction mixture.

Examples of polyglycerol fatty acid esters usable as an emulsifier in the present invention include an ester of a polyglycerol having an average degree of polymerization of from 2 or more (preferably from 6 to 15, more preferably from 8 to 10) and a fatty acid having 8 to 18 carbon atoms (e.g., caprylic acid, capric acid, lauric acid, myristic acid, palmitic acid, stearic acid, oleic acid, or linolic acid). Preferable examples of polyglycerol fatty acid esters include hexaglycerol monooleate ester, hexaglycerol monostearate ester, hexaglycerol monopalmitate ester, hexaglycerol monomyristate ester, hexaglycerol monolaurate ester, decaglycerol monooleate ester, decaglycerol monostearate ester, decaglycerol monopalmitate ester, decaglycerol monomyristate ester, and decaglycerol monolaurate ester. The polyglycerol fatty acid ester may be used singly or as a mixture of two or more thereof. Examples of a commercially available polyglycerol fatty acid esters include products of Nikko Chemicals Co., Ltd., such as NIKKOL DGMS, NIKKOL DGMO-CV, NIKKOL DGMO-90V, NIKKOL DGDO, NIKKOL DGMIS, NIKKOL DGTIS, NIKKOL TETRAGLYN 1-SV, NIKKOL TETRAGLYN 1-O, NIKKOL TETRAGLYN 3-S, NIKKOL TETRAGLYN 5-S, NIKKOL TETRAGLYN 5-O, NIKKOL HEXAGLYN 1-L, NIKKOL HEXAGLYN 1-M, NIKKOL HEXAGLYN 1-SV, NIKKOL HEXAGLYN 1-O, NIKKOL HEXAGLYN 3-S, NIKKOL HEXAGLYN 4-B, NIKKOL HEXAGLYN 5-S, NIKKOL HEXAGLYN 5-O, NIKKOL HEXAGLYN PR-15, NIKKOL DECAGLYN 1-L, NIKKOL DECAGLYN 1-M, NIKKOL DECAGLYN 1-SV, NIKKOL DECAGLYN 1-50SV, NIKKOL DECAGLYN 1-ISV, NIKKOL DECAGLYN 1-O, NIKKOL DECAGLYN 1-OV, NIKKOL DECAGLYN 1-LN, NIKKOL DECAGLYN 2-SV, NIKKOL DECAGLYN 2-ISV, NIKKOL DECAGLYN 3-SV, NIKKOL DECAGLYN 3-OV, NIKKOL DECAGLYN 5-SV, NIKKOL DECAGLYN 5-HS, NIKKOL DECAGLYN 5-IS, NIKKOL DECAGLYN 5-OV, NIKKOL DECAGLYN 5-O-R, NIKKOL DECAGLYN 7-S, NIKKOL DECAGLYN 7-O, NIKKOL DECAGLYN 10-SV, NIKKOL DECAGLYN 10-IS, NIKKOL DECAGLYN 10-OV, NIKKOL DECAGLYN 10-MAC, and NIKKOL DECAGLYN PR-20 (tradenames); products of Mitsubishi-Kagaku Foods Corporation, such as RYOTO POLYGLY ESTER L-10D, L-7D, M-10D, M-7D, P-8D, S-28D, S-24D, SWA-20D, SWA-15D, SWA-10D, O-50D, O-15D, B-100D, B-70D, and ER-60D (tradenames); products of TAIYO KAGAKU CO., LTD., such as SUN SOFT Q-17UL, SUN SOFT Q-14S, and SUN SOFT A-141C (tradenames); and products of Riken Vitamin Co., Ltd., such as POEM DO-100 and POEM J-0021 (tradenames).

In sorbitan fatty acid esters usable in the emulsion composition of the present invention, the fatty acid has preferably 8 or more carbon atoms, and more preferably 12 or more carbon atoms. Preferable examples of sorbitan fatty acid esters include sorbitan monocaprylate, sorbitan monolaurate, sorbitan monostearate, sorbitan sequistearate, sorbitan tristearate, sorbitan isostearate, sorbitan sesquiisostearate, sorbitan oleate, sorbitan sesquioleate, and sorbitan triolate. The sorbitan fatty acid ester may be used singly or as a mixture two or more thereof. Examples of commercially available sorbitan fatty acid esters include products of Nikko Chemicals Co., Ltd., such as NIKKOL SL-10, SP-10V, SS-10V, SS-10MV, SS-15V, SS-30V, SI-10RV, SI-15RV, SO-10V, SO-15MV, SO-15V, SO-30V, SO-10R, SO-15R, SO-30R, and SO-15EX (tradenames); and products of Dai-Ichi Kogyo Seiyaku Co., Ltd., such as SORGEN 30V, 40V, 50V, 90, and 110 (tradenames).

In sucrose fatty acid esters usable in the emulsion composition of the present invention, the fatty acid has preferably 12 or more carbon atoms, and more preferably 12 to 20 carbon atoms. Preferable examples of sucrose fatty acid esters include sucrose dioleate, sucrose distearate, sucrose dipalmitate, sucrose dimyristate, sucrose dilaurate, sucrose monooleate, sucrose monostearate, sucrose monopalmitate, sucrose monomyristate, and sucrose monolaurate. In the present invention, the sucrose fatty acid ester may be used singly or as a mixture of two or more thereof. Examples of a commercially-available sucrose fatty acid ester include products of Mitsubishi-Kagaku Foods Corporation, such as RYOTO SUGAR ESTER S-070, S-170, S-270, S-370, S-370F, S-570, S-770, S-970, S-1170, S-1170F, S-1570, S-1670, P-070, P-170, P-1570, P-1670, M-1695, 0-170, 0-1570, OWA-1570, L-195, L-595, L-1695, LWA-1570, B-370, B-370F, ER-190, ER-290, and POS-135 (tradenames); and products of Dai-Ichi Kogyo Seiyaku Co., Ltd., such as DK ester SS, F160, F140, F110, F90, F70, F50, F-A50, F-20W, F-10, and F-A10E, COSMELIKE B-30, S-10, S-50, S-70, S-110, S-160, S-190, SA-10, SA-50, P-10, P-160, M-160, L-10, L-50, L-160, L-150A, L-160A, R-10, R-20, O-10, and 0-150 (tradenames).

A nonionic surfactant in the present invention has an HLB of preferably from 10 to 16 for the improvement in dispersibility, and more preferably from 12 to 16 from the viewpoint of stability of an emulsion.

The addition amount of the surfactant varies depending on the form of the emulsion composition. In order to obtain a water dispersion in which fine O/W emulsion particles are dispersed, the addition amount of the surfactant is preferably from 0.1 mass% to 50 mass%, more preferably from 0.5 to 20 mass%, and still more preferably from 1 mass% to 15 mass%, with respect to the water dispersion. By adjusting the addition amount to 0.1 mass% or more, an emulsion having a smaller particle diameter can be obtained and also the stability of the emulsion can be sufficiently secured. By adjusting the addition amount to 50 mass% or lower, foaming of the emulsion can be adjusted to a suitable range.

### <Glycerol>

In the present invention, from the viewpoint of obtaining a water dispersion in which fine O/W emulsion particles are dispersed, the water dispersion preferably contains glycerol, whereby the average particle diameter of the emulsion particles in the water dispersion can be further reduced and stably maintained small over a long period of time.

In this case, the content of glycerol is preferably from 10 mass% to 60 mass%, more preferably from 20 mass% to 55 mass%, and sill more preferably from 30 mass% to 50 mass%, with respect to the total mass of the water dispersion from the viewpoint of dispersion stability and antiseptic properties.

### <Other components>

To the emulsion composition of the present invention, components used for common skin preparations for external use and/or cosmetics may be added in addition to the above-mentioned components. Specific examples thereof include oils and fats (e.g., natural animal oils and fats, natural vegetable oils and fats, semisynthetic oils and fats, hydrocarbon oils, higher fatty acids, ester oils, silicone oils, and fluorine-containing oils), gelling agents, metallic soaps, surfactants (anionic surfactants, cationic surfactants, and amphoteric surfactants), powder other than those mentioned above (e.g., inorganic powder, organic powder, or pigment), alcohols (e.g., higher alcohols, polyhydric alcohols, and sterols), water-soluble polymers (animal-derived water-soluble polymers, plant-derived water-soluble polymers, microorganism-derived water-soluble polymers, and synthetic water-soluble polymers), film-forming ingredients, resins, antiseptic agents, antibacterial agents, flavors, essential oils, salts, water (purified water, hot spring water, and deep sea water), PH adjustors, refrigerants, moisturizers, anti-inflammatory agents, whitening agents, cell activators, rough-skin ameliorating agents, circulation accelerators, skin astringents, antiseborrheic agents, amino acids, nucleic acid related compounds, enzymes, hormones, inclusion compounds, plant extracts, extracts derived from animals, and extracts derived from microorganisms.

### <Form of an emulsion composition >

There is no limitation on the form of the emulsion composition of the present invention insofar as the oil phase and the aqueous phase are clearly divided. For example, various forms, such as a water dispersion, a milky lotion, a multilayer, a paste, and a gel, may be selected. From the viewpoint of use feeling, it is preferable for the emulsion composition of the present invention to be in the form of milky lotion, multilayer, paste, or gel.

The emulsion composition of the present invention may be prepared by blending the respective components described above in a general base material that is selected according to the form of the emulsion composition, according to a generally used method that is selected in accordance with the form of the emulsion composition under generally employed blending conditions.

There is no limitation on the volume average particle diameter of the emulsion particles of the emulsion composition in the present invention. When the above-mentioned component (B) is contained in the O/W emulsion particles dispersed in the aqueous phase, the volume average particle diameter of the emulsion particles is preferably from 1 nm to 300 nm from the viewpoint of particle stability and transparency, and, from the viewpoint of the transparency of the aqueous phase, is more preferably 130 nm or less, and is most preferably 90 nm or less.

The volume average particle diameter can be measured with a commercially available particle size distribution analyzer or the like. As a method of measuring the particle diameter of an emulsion, an optical microscope method, a confocal laser scanning microscope method, an electron microscope method, an atomic force microscope method, a static light scattering method, a laser diffraction method, a dynamic light scattering method, a centrifugal method, an electrical pulse measuring method, a chromatographic method, an ultrasonic attenuation method, and the like are known, and apparatuses corresponding to the respective principles are commercially available.

It is preferable to use the dynamic light scattering method for the measurement of the volume average particle diameter of the emulsion in the present invention, in terms of measurable range of the volume average particle diameter and ease of measurement in the present invention. Examples of a commercially available measuring apparatuses using the dynamic light scattering method include NANOTRUCK UPA (tradename, Nikkiso Co., Ltd.), a dynamic light scattering type particle size distribution measuring apparatus LB-550 (tradename, Horiba, Ltd.), and a fiber-optics particle analyzer FPAR-1000 (tradename, Otsuka Electronics).

The volume average particle diameter of an O/W emulsion mentioned in the invention refers to a value measured at 25°C with a fiber-optics particle analyzer FPAR-1000 (manufactured by Otsuka Electronics).

The volume average particle diameter is measured by diluting the sample with pure water such that the concentration of the oil phase components falls within the range of from 0.1 to 1 mass% and performing a measurement using a glass tube. The volume average particle diameter can be obtained as a diameter at which an accumulated volume reaches (50%) when measured with a dispersion-medium refractive index of 1.3313 (pure water) and a dispersion medium viscosity of 0.8846 cp (pure water).

The particle diameter of the emulsion composition can be adjusted by factors such as stirring conditions (shearing force, temperature, and pressure) in the production method and the ratio of the oil phase to the aqueous phase, besides the components of the emulsion composition.

### <Method of producing an emulsion composition>

There is no limitation on a method of producing an emulsion composition. For example, a production method is preferable which includes (I) dissolving a water-soluble emulsifier in an aqueous media (water or the like) to obtain an aqueous phase, (II) mixing and dissolving an oil-soluble component, phospholipid, etc., to obtain an oil phase, and (III) mixing the aqueous phase and the oil phase under stirring, and emulsifying and dispersing the resultant to obtain an emulsion composition.

The oil phase/aqueous phase ratio (by mass) in the dispersing emulsification may be suitably selected according to the desired form of the emulsion composition.

For example, in order to obtain an emulsion composition containing fine O/W emulsion particles or in order to preliminarily prepare a water dispersion of fine O/W emulsion particles that is to be added to the aqueous phase of an emulsion composition, the oil phase/aqueous phase ratio (by mass) in the dispersing emulsification is not particularly limited. However, a lower oil phase/aqueous phase ratio (by mass) generally leads to a smaller particle diameter. When the oil phase/aqueous phase ratio (mass) is excessively small, however, the concentration of an active ingredient becomes low and a problem in practical use may occur, and/or the concentration of an emulsifier decreases and the emulsion stability of the emulsion composition may be deteriorated.

From the above viewpoints, the oil phase/aqueous phase ratio (mass%) of an O/W emulsion composition is preferably in the range of from 0.1/99.9 to 50/50, more preferably from 0.5/99.5 to 30/70, and still more preferably from 1/99 to 20/80.

Here, the proportion of water in the O/W emulsion composition is preferably 80 mass% or more, and more preferably 85 mass% or more, from the viewpoint of obtaining smaller emulsion particles.

The dispersing emulsification may be performed by one emulsification step. However, it is preferable to perform two or more emulsification steps from the viewpoint of obtaining uniform and fine emulsion particles.

Specifically, it is particularly preferable to use two or more kinds of emulsification apparatuses in combination; for example, a method may be employed which includes performing emulsification with a high pressure homogenizer or the like, in addition to a one-step emulsification operation of performing emulsification with a general emulsification apparatus utilizing a shearing action (e.g., a stirrer, an impeller stirrer, a homomixer, or a continuous-flow shearing apparatus). When a high pressure homogenizer is used, the uniformity of the fine particles (liquid droplets) in the emulsion may be improved. Furthermore, the above operations may be repeated plural times so as to make the particle sizes of liquid droplets more uniform.

There is no particular limitation on the temperature condition at the time of performing the dispersin emulsification in the present invention. The temperature is preferably from 10 to 100°C from the viewpoint of stability of a functional oil component. A suitable temperature range may be selected in consideration of the melting point or the like of the functional oil component to be used.

Examples of the high pressure homogenizer include a chamber high pressure homogenizer having a chamber in which a flow path of treatment liquid is fixed and a homogenizing-valve high pressure homogenizer having a homogenizing valve. Among the above, in the homogenizing-valve high pressure homogenizer, the width of the flow path of the treatment liquid can be easily adjusted and the pressure and flow rate at the time of operation can be freely adjusted, whereby a wide operation range is achieved. Thus, the homogenizing-valve high pressure homogenizer is preferable particularly for the method of producing the emulsion composition of the present invention.

When an ultra high pressure is required, the chamber high pressure homogenizer can be preferably used in view of the easiness in producing a pressure increasing mechanism, though the degree of freedom of operation is lower.

Examples of the chamber type high pressure homogenizer include a MICROFLUIDIZER (tradename, manufactured by Microfluidics Inc.), a NANOMIZER (tradename, manufactured by Yoshida Kikai Co., Ltd.), and an ALTIMIZER (tradename, manufacture by Sugino Machine Co., Ltd.).

Examples of the homogenizing valve type high pressure homogenizer include a gaulin type homogenizer (manufactured by APV), a Lannier type homogenizer (manufactured by Lannier), a high pressure homogenizer (manufactured by Niro Soavi), a homogenizer (manufactured by Sanwa Machinery Trading Co., Ltd.), a high pressure homogenizer (manufactured by Izumi Food Machinery Co., Ltd.), and an ultra high pressure homogenizer (manufactured by IKA).

In the present invention, treatment pressure of the high pressure homogenizer is preferably 50 MPa or more, more preferably from 50 to 250 MPa, and still more preferably from 100 to 250 MPa.

The emulsion liquid obtained by subjecting the composition to a dispersing emulsification, is preferably cooled by being passed through a condenser immediately after passing the chanber (for example within 30 seconds, preferably within 3 seconds, from passing the chamber) from the viewpoint of maintaining the particle diameter of the dispersed particles.

An O/W emulsion composition in which emulsion particles containing an oil component are dispersed in an aqueous medium is obtained by the above-described process.

When the emulsion composition of the present invention is an emulsion composition containing fine O/W emulsion particles in an aqueous phase, the emulsion composition can be prepared by adding a preliminarily-prepared water dispersion of O/W emulsion particles to the aqueous phase of the emulsion composition. The water dispersion can be prepared in a manner similar to the preparation of the O/W emulsion composition described above.

When the water dispersion is blended in the aqueous phase of the emulsion composition of the present invention, the content of the water dispersion is preferably from 0.0001 mass% to 20 mass%, and more preferably from 0.001 mass% to 10 mass%, based on the total mass of the aqueous phase components of the emulsion composition from the viewpoint of effectively demonstrating the function of the oil-soluble component contained in the O/W emulsion particles in the water dispersion.

As a result, an emulsion composition can be obtained in which O/W emulsion particles of the water dispersion are dispersed in the aqueous phase separately from the oil phase (emulsion particles) of the emulsion composition.

In the emulsion composition of the present invention, the functions of the active oxygen remover and/or anti-oxidizing agent can be stably maintained over a long period of time even when a UV scattering agent is used together with the active oxygen remover and/or anti-oxidizing agent. Thus, the emulsion composition of the present invention can be preferably used for applications in which functions of the active oxygen remover or the anti-oxidizing agent are effective, e.g., skin external preparations and cosmetics.

### EXAMPLES

### [Example 1]

### (1) Preparation of water dispersion (i)-a and water dispersion (i)-b

The respective components shown in Table 1 were dissolved over 1 hour under heating at 70°C to obtain an aqueous composition.

Moreover, the respective components shown in Table 2 were dissolved over 1 hour under heating at 70°C to obtain an oil phase composition.

**Table 1**

| | |
|---|---|
| Sucrose stearate ester (HLB = 16) | 13 g |
| Decaglyceryl monooleate (HLB=12) | 25 g |
| Glycerol | 500 g |
| Pure water | 322 g |

| | |
|---|---|
| Sucrose stearate ester: Ryoto Sugar Ester S-1670 (HLB = 16, tradename) manufactured by Mitsubishi-Kagaku Foods Corporation Decaglyceryl monooleate: NIKKOL DECAGLYN 1-O (HLB = 12, tradename) manufactured by Nikko Chemicals Co., Ltd. | |

**Table 2**

| | |
|---|---|
| Haematococcus algae extract (containing 20 mass% of astaxanthins) | 40 g |
| Mix Tocopherol | 10 g |
| Lecithin (derived from soybean) | 90 g |

| | |
|---|---|
| Haematococcus algae extract: ASTOTS-S (tradename) manufactured by TAKEDA SHIKI Co., Ltd. Mix Tocopherol: Riken E oil 800 (tradename) manufactured by Riken Vitamin Co., Ltd. Lecithin (derived from soybean): LECION P (tradename) manufactured by Riken Vitamin Co., Ltd. | |

The aqueous phase was stirred (10,000rpm) with a homogenizer (Vacuum emulsifying apparatus PVQ-1D model (tradename), manufactured by Mizuho Kogyo Kabushiki Kaisha) while the temperature of the aqueous phase was maintained at 70°C. Then, the oil phase composition was added thereto to obtain an emulsion. The obtained emulsion was subjected to high pressure emulsification at 40°C and a pressure of 200 MPa using an ALTIMIZER HJP-25005 (tradename, manufactured by Sugino Machine Co., Ltd.).

Thereafter, the resultant was filtered through a microfilter having an average pore diameter of 1 µm to obtain a water dispersion (i)-a containing an astaxanthin.

A water dispersion (i)-b containing astaxanthin was similarly produced except adding 90 g of pure water in place of 90 g of lecithin in the oil phase composition.

### (2) Production of aqueous compositions (ii)-a to (ii)-l

Each of the oil phase component and aqueous phase component shown below was warmed to 70 to 80°C, and then was treated for 5 minutes at 5,000 rpm with a homomixer so as to achieve uniform dissolution or dispersion. The oil phase component was added to the aqueous phase component, and stirred at 6,000 rpm for 10 minutes with a homomixer while the mixture was maintained at a temperature of 80 °C. Thereafter, the mixture was cooled under paddle stirring. The stirring was stopped when the temperature had cooled to within a range of from 30 to 35°C, and allowed to stand for 10 minutes to obtain emulsion compositions (ii)-a to (ii)-l.

| (Oil phase component) | mass % |
|---|---|
| Polyglycerol/sodium stearoyl lactate mixture | 2.5 |
| glyceryl tri(caprylate/caprate) | 3.0 |
| Behenyl alcohol | 2.5 |
| Squalane | 4.0 |
| Glyceryl tri-2-ethylhexanoate | 3.0 |
| 2-ethylhexyl para-methoxycinnamate | 4.0 |
| Octocrylene | 2.6 |
| Diethylaminohydroxybenzoyl hexyl benzoate | 0.5 |
| Decamethyl cyclopentasiloxane | 1.0 |
| Haematococcus algae extract | (as indicated in Table 3) |
| UV scattering agent (as indicated in Table 3) | (as indicated in Table 3) |

| (Aqueous phase component) | mass% |
|---|---|
| Antiseptic agent | Appropriate amount |
| 1,3-butylene glycol | 5.0 |
| Xanthan gum | 0.3 |
| Astaxanthin-containing water dispersion (indicated in Table 3) | (as indicated in Table 3) |
| UV scattering agent (as indicated in Table 3) | (as indicated in Table 3) |
| Purified water | (such an amount as to adjust the total amount to 100.0 mass%) |

### Measurement of physical property values

### (1) Change with time at 40°C

A sample was put in a light-shielding container, and the lid is closed. The container was placed in a 40°C thermostat, and then stored for 60 days. The results are shown in Table 3.

The absorbance was measured based on a reflection spectrum, using an integrating sphere and a UV-VISIBLE spectrum photometer UV-2550 (manufactured by Shimadzu Corp.). The absorption spectrum was determined based on the spectrum of the total reflection, and the absorption at 478 nm was used for determining the absorbance. For the purpose of the evaluation, the residual ratio determined from ratio of the absorbance after the storage in the thermostat to the absorbance immediately after the preparation of the composition is considered to represent the fading level of the dispersion liquid. The criteria for the evaluation are as follows:
A : The absorbance residual ratio is 70% or more (a satisfactory level in which no changes can be recognized when the emulsion composition is applied to a palm)
B: The absorbance residual ratio is 50% or more but 70 % or less (a commercially acceptable level though changes can be recognized when the emulsion composition is applied to a palm)
C : The absorbance residual ratio is lower than 50% (an unacceptable level)

The volume average particle diameter of each of the astaxanthin-containing water dispersions (i)-a and (i)-b in the emulsion composition was measured at 20°C using a dynamic light scattering type particle size distribution measuring apparatus LB-550 (manufactured by Horiba, Ltd.), and was found to be 70 nm and 350 nm, respectively.

**Table 3**

| | | Type | (ii)-a | (ii)-b | (ii)-c | (ii)-d | (ii)-e | (ii)-f | (ii)-g | (ii)-h | (ii)-i | (ii)-j | (ii)-k | (ii)-l |
|---|---|---|---|---|---|---|---|---|---|---|---|---|---|---|
| Oil phase | Haematococcus algae extract | ASTOTS-S | 0.0075 | 0.0075 | - | - | 0.0075 | 0.0075 | - | - | - | - | - | - |
| | UV scattering agent | Silicon-treated titanium oxide | 2 | - | - | - | - | - | 2 | - | 1 | 1 | - | - |
| | | Silica-treated zinc oxide | - | 2 | - | - | - | - | - | 2 | 1 | 1 | - | - |
| | | Talc | - | - | - | - | - | - | - | - | - | - | 2 | - |
| | | PMMA spherical particles (2 µm) | - | - | - | - | - | - | - | - | - | - | - | 1 |
| Aqueous phase | Water dispersion containing Haematococcus algae extract | (i)-a | - | - | 0.15 | 0.15 | - | - | 0.15 | 0.15 | 0.15 | - | 0.15 | 0.15 |
| | | (i)-b | - | - | - | - | - | - | - | - | - | 0.15 | - | - |
| | UV scattering agent | Silicon-treated titanium oxide | - | - | 2 | - | 2 | - | - | - | - | - | - | - |
| | | Silica-treated zinc oxide | - | - | - | 2 | - | 2 | - | - | - | - | - | - |
| | | Evaluation of stability at 40°C for 2M | C | C | C | C | B | B | A | A | A | B | A | A |
| | | Residual ratio | 40% | 45% | 35% | 40% | 60% | 62% | 85% | 89% | 88% | 65% | 80% | 85% |
| | | Remarks | Comp. Example | Comp. Example | Comp. Example | Comp. Example | Invention | Invention | Inven tion | Inven tion | Inven tion | Inven tion | Inven tion | Inven tion |

As shown in Table 3, the compositions of Examples of the present invention exhibited less fading than Comparative Examples, and the components in the composition of Examples, such as astaxanthin, are stable. Thus, it is revealed that the emulsion compositions of Examples of the present invention can satisfactorily maintain the active oxygen remover and the anti-oxidizing agent and are excellent in stability even when a UV scattering agent is used together.

## Claims

1. An emulsion composition comprising a UV scattering agent and at least one of an active oxygen remover or an anti-oxidizing agent, wherein the at least one of an active oxygen remover or an anti-oxidizing agent is contained in O/W emulsion particles dispersed in an aqueous phase of the emulsion composition, the UV scattering agent is added to an oil phase of the emulsion composition which is different from the O/W emulsion particles wherein the active oxygen remover is a carotenoid and the anti-oxidizing agent is at least one selected from the group consisting of a vitamin C, derivatives of vitamin C, and salts of vitamin C, selected from the group consisting of ascorbic acid, L-ascorbic acid, L-ascorbyl palmitate, L-ascorbyl dipalmitate, L-ascorbyl isopalmitate, L-ascorbyl diisopalmitate, L-ascorbyl tetraisopalmitate, L-ascorbyl stearate, L-ascorbyl distearate, L-ascorbyl isostearate, L-ascorbyl diisostearate, L-ascorbyl myristate, L-ascorbyl dimyristate, L-ascorbyl isomyristate, L-ascorbyl diisomyristate, L-ascorbyl oleate, L-ascorbyl dioleate, L-ascorbyl 2-ethyl hexanoate, sodium L-ascorbyl 2-phosphate, potassium L-ascorbyl phosphate, magnesium L-ascorbyl phosphate, calcium L-ascorbyl phosphate, aluminum L-ascorbyl phosphate, sodium L-ascorbyl sulfate, potassium L-ascorbyl sulfate, magnesium L-ascorbyl sulfate, calcium L-ascorbyl sulfate, aluminum L-ascorbyl sulfate, sodium L-ascorbate, potassium L-ascorbate, magnesium L-ascorbate, calcium L-ascorbate, and aluminum L-ascorbatea vitamin E, derivatives of vitamin E, and salts of vitamin E, selected from the list consisting of: tocopherol, dl-α (β, γ)-tocopherol, dl-α-tocopherol acetate, dl-α-tocopherol nicotinate, dl-α-tocopherol linolate, and dl-α-tocopherol succinate, and ubiquinones.

2. The emulsion composition according to claim 1, wherein the at least one of an active oxygen remover or an anti-oxidizing agent comprises at least one active oxygen remover and at least one anti-oxidizing agent.

3. The emulsion composition according to claim 1, wherein the UV scattering agent is at least one selected from titanium oxide or zinc oxide.

4. The emulsion composition according to claim 1, further comprising a UV absorber.

5. The emulsion composition according to claim 1, further comprising a phospholipid.

6. The emulsion composition according to claim 5, wherein the phospholipid is lecithin.

7. The emulsion composition according to claim 1, wherein the carotenoid is astaxanthin.

8. The emulsion composition according to claim 1, further comprising a surfactant selected from the group consisting of polyglycerol fatty acid esters and sucrose fatty acid esters.

## Patentansprüche

1. Emulsionszusammensetzung enthaltend ein UV-streuendes Mittel und ein aktiven Sauerstoff entfernendes Mittel und/oder ein antioxidierendes Mittel, wobei das aktiven Sauerstoff entfernende Mittel und/oder das antioxidierende Mittel in O/W Emulsionspartikeln enthalten ist, die in einer wässrigen Phase der Emulsionszusammensetzung dispergiert sind, wobei das UV-streuende Mittel zu einer Ölphase der Emulsionszusammensetzung zugegeben wird, die sich von den O/W Emulsionspartikeln unterscheidet, wobei das aktiven Sauerstoff entfernende Mittel ein Carotinoid ist und das antioxidierende Mittel mindestens eines, ausgewählt aus der Gruppe bestehend aus einem Vitamin C, Derivaten von Vitamin C und Salzen von Vitamin C, ausgewählt aus der Gruppe bestehend aus Ascorbinsäure, L-Ascorbinsäure, L-Ascorbylpalmitat, L-Ascorbyldipalmitat, L-Ascorbylisopalmitat, L-Ascorbyldiisopalmitat, L-Ascorbyltetraisopalmitat, L-Ascorbylstearat, L-Ascorbyldistearat, L-Ascorbylisostearat, L-Ascorbyldiisostearat, L-Ascorbylmyristat, L-Ascorbyldimyristat, L-Ascorbylisomyristat, L-Ascorbyldiisomyristat, L-Ascorbyloleat, L-Ascorbyldioleat, L-Ascorbyl-2-ethylhexanoat, Natrium-L-ascorbyl-2-phosphat, Kalium-L-ascorbylphosphat, Magnesium-L-ascorbylphosphat, Calcium-L-ascorbylphosphat, Aluminium-L-ascorbylphosphat, Natrium-L-ascorbylsulfat, Kalium-L-ascorbylsulfat, Magnesium-L-ascorbylsulfat, Calcium-L-ascorbylsulfat, Aluminium-L-ascorbylsulfat, Natrium-L-ascorbat, Kalium-L-ascorbat, Magnesium-L-ascorbat, Calcium-L-ascorbat und Aluminium-L-ascorbat, einem Vitamin E, Derivaten von Vitamin E und Salzen von Vitamin E, ausgewählt aus der Liste bestehend aus: Tocopherol, dl-α (β, γ)-Tocopherol, dl-α-Tocopherolacetat, dl-α-Tocopherolnicotinat, dl-α-Tocopherollinolat und dl-α-Tocopherolsuccinat und Ubichinonen, ist.

2. Emulsionszusammensetzung nach Anspruch 1, wobei das aktiven Sauerstoff entfernende Mittal und/oder das antioxidierende Mittel mindestens ein aktiven Sauerstoff entfernendes Mittel und mindestens ein antioxidierendes Mittel umfasst.

3. Emulsionszusammensetzung nach Anspruch 1, wobei das UV-streuende Mittel mindestens eines, ausgewählt aus Titanoxid und Zinkoxid ist.

4. Emulsionszusammensetzung nach Anspruch 1, ferner enthaltend einen UV-Absorber.

5. Emulsionszusammensetzung nach Anspruch 1, ferner enthaltend ein Phospholipid.

6. Emulsionszusammensetzung nach Anspruch 5, wobei das Phospholipid Lecithin ist.

7. Emulsionszusammensetzung nach Anspruch 1, wobei das Carotinoid Astaxanthin ist.

8. Emulsionszusammensetzung nach Anspruch 1, ferner enthaltend ein Tensid, ausgewählt aus der Gruppe bestehend aus Polyglycerinfettsäureestern und Saccharosefettsäureestern.

## Revendications

1. Composition d'émulsion, comprenant un agent de diffusion des UV et au moins un élément parmi un agent d'élimination à l'oxygène actif ou un agent antioxydant, dans lequel le au moins un élément parmi un agent d'élimination à l'oxygène actif ou un agent antioxydant est contenu dans des particules d'émulsion d'huile dans l'eau dispersées dans une phase aqueuse de la composition d'émulsion, l'agent de diffusion des UV est ajouté à une phase huileuse de la composition d'émulsion, laquelle est différente des particules d'émulsion d'huile dans l'eau, dans lequel l'agent d'élimination à l'oxygène actif est un caroténoïde, et l'agent antioxydant est au moins un élément sélectionné parmi le groupe consistant en une vitamine C, des dérivés de vitamine C, et des sels de vitamine C, sélectionnés parmi le groupe consistant en l'acide ascorbique, l'acide L-ascorbique, le palmitate de L-ascorbyle, le dipalmitate de L-ascorbyle, l'isopalmitate de L-ascorbyle, le diisopalmitate de L-ascorbyle, le tétraisopalmitate de L-ascorbyle, le stéarate de L-ascorbyle, le distéarate de L-ascorbyle, l'isostéarate de L-ascorbyle, le diisostéarate de L-ascorbyle, le myristate de L-ascorbyle, le dimyristate de L-ascorbyle, l'isomyristate de L-ascorbyle, le diisomyristate de L-ascorbyle, l'oléate de L-ascorbyle, le dioléate de L-ascorbyle, l'hexanoate de L-ascorbyl-2-éthyle, le 2-phosphate de L-ascorbyle de sodium, le phosphate de L-ascorbyle de potassium, le phosphate de L-ascorbyle de magnésium, le phosphate de L-ascorbyle de calcium, le phosphate de L-ascorbyle d'aluminium, le sulfate de L-ascorbyle de sodium, le sulfate de L-ascorbyle de potassium, le sulfate de L-ascorbyle de magnésium, le sulfate de L-ascorbyle de calcium, le sulfate de L-ascorbyle d'aluminium, le L-ascorbate de sodium, le L-ascorbate de potassium, le L-ascorbate de magnésium, le L-ascorbate de calcium, et le L-ascorbate d'aluminium, une vitamine E, des dérivés de vitamine E, et des sels de vitamine E, sélectionnés parmi la liste consistant en le tocophérol, le di-α(β,γ)-tocophérol, l'acétate de di-α-tocophérol, le nicotinate de di-α-tocophérol, le linolate de di-α-tocophérol, et du succinate de di-α-tocophérol, et des ubiquinones.

2. Composition d'émulsion selon la revendication 1, dans laquelle le au moins un élément parmi un agent d'élimination à l'oxygène actif ou un agent antioxydant comprend au moins un élément parmi un agent d'élimination à l'oxygène actif et au moins un agent antioxydant.

3. Composition d'émulsion selon la revendication 1, dans laquelle l'agent de diffusion des UV est au moins un élément sélectionné parmi l'oxyde de titane et l'oxyde de zinc.

4. Composition d'émulsion selon la revendication 1, comprenant en outre un agent absorbant les UV.

5. Composition d'émulsion selon la revendication 1, comprenant en outre un phospholipide.

6. Composition d'émulsion selon la revendication 5, dans laquelle le phospholipide est de la lécithine.

7. Composition d'émulsion selon la revendication 1, dans laquelle le caroténoïde est de l'astaxanthine.

8. Composition d'émulsion selon la revendication 1, comprenant en outre un surfactant sélectionné parmi le groupe consistant en des esters polyglycéroliques d'acides gras et des esters d'acides gras de sucrose.
